# EUROPEAN PATENT APPLICATION

(11) **EP 2 865 356 A1**
(43) Date of publication of application: **29.04.2015**
(21) Application number: 14190163.7
(22) Date of filing: 23.10.2014
(51) Int. Cl.: A61F 2/58, A61F 2/68, A61F 2/70

(54) **Hybrid prosthetic hand**

(30) Priority: 23.10.2013 US 201314061646
(71) Applicant: Schoen, Marco P., Pocatello, ID 83204 (US); Scott, Kurt W., Blackfoot, ID 83221 (US)
(72) Inventor: Schoen, Marco P., Pocatello, ID 83204 (US); Scott, Kurt W., Blackfoot, ID 83221 (US)
(74) Representative: Dixon, Philip Matthew

(57) **Abstract**

A hybrid prosthetic hand is detailed which is controlled via input from both sEMG signals, as well as mechanical control from the elbow and shoulder of an amputee. The device is equipped with mechanical fingers, which are driven by electrical motors, and controlled via microcontrollers. The mechanical fingers are designed to form a variety of shapes and provide variable force in accordance with the contextual desires of the amputee, which are conveyed to the device primarily through the movement of the shoulder and/or elbow of the amputee. The instructions sent to the mechanical fingers of the device by the shoulder or elbow is augmented by instructions provided via sEMG signals.

## Description

### FIELD OF THE PRESENT INVENTION

The present invention is in the technical field of prosthetic devices. More particularly, the present invention is in the technical field of hand prosthetic devices using shoulder harness and electromyographic (EMG) based activation and control.

### BACKGROUND OF THE PRESENT INVENTION

Current statistics about trans-radial amputation indicates upper limb prosthetics is in high demands. A lot of effort has been placed on research dealing with advanced prosthetic devices. However, up to date there are no prosthetic devices available that mimic the full functionality of a human hand. There are multiple types of hand prostheses. In most cases these devices are controlled using a shoulder harness that allows the operator to capture shoulder or elbow movements and translate these into a mechanical opening and closing of a hook or clamp that is used to give some ability back to the amputee. Another type of prosthetic hand device is based on measuring surface Electromyographic (sEMG) signals to initiate the actuation of the prosthetic device. Since sEMG signals are spatially distributed, the sEMG probes pick up signals from other motor units stemming from different muscle groups. This phenomenon is called crosstalk, which is a major cause for the difficulty in interpreting the intended hand/finger motion. EMG signals are generated by the simultaneous firing of several motor units during muscle contraction. Before reaching the skin surface, the EMG signal passes through numerous layers of tissues, which leads to noise and interference in the signal acquisition. The random nature of the sEMG signal represents an added complexity in studying it. All of these issues make it rather complex to distinguish the content of the sEMG signal against noise and interference.

Prosthetic devices have been developed with the aim of matching the human hand in terms of dexterity and adaptation capabilities. Prosthetic hands are often designed to equip a dexterous manipulator for pick-and-place tasks or full mechanical designs that use other motion of the body - typically shoulder or elbow motion - to create a gripping motion in prosthetic devices. Both types of devices have been shown to give some functionality back to an amputee. In manipulator pick-and-place devices, the prosthetic device looks and feels like the human hand and can create human hand like function. However, training and fitting of these devices is cumbersome and often times the amputee will part from using the device in as little as two years. Full mechanical design prosthetics provide robust designs that simplify the human hand function down to the simplicity of a simple hook that has the ability to pinch an object. These designs have become the fall back choice for most amputees.

The devise that is described in this application is a hybrid version of the systems described above. It has both mechanical actuation functions, such as typical mechanical prosthetic devices possess with the dexterous manipulator, and actuation function controlled by the use of sEMG signals. The present invention has the ability to control hand motion by both types of actuation. This allows the amputee to us the mechanical controls/actuation to help train mussels when actuating the prosthetic with sEMG signals. It also allows the amputee to choose what type of actuation (sEMG, mechanical, or both at the same time) he or she desires for a particular task, making the system adaptive to the user.

### SUMMARY OF THE PRESENT INVENTION

The present invention is a hybrid prosthetic hand capable of controlling the motion set of a prosthetic device through sEMG signals as well as through the motion of the respective shoulder and elbow of the prosthetic hand user.

The mechanical functions of the present invention are controlled by either a shrugging shoulder motion, elbow straitening or collapsing motion, or both. The forces provided by these motions are transferred to hand using a Bowden cable design. The operator can then chose what this motion will do in the hand function, allowing the system to adapt to the amputee's personal ability and desired task.

The second type for controlling the prosthetic device is given by using the sEMG signals. In this mode, the present invention utilizes a hierarchical control structure, where classification of sEMG signals are used to infer the general motion set, and sEMG signals-motion as well as sEMG signal-force models are used to control the intricate motion of individual joints of the prosthetic device.

A third option of controlling the prosthetic device is given by evoking both approaches described above at the same time as a hybrid mode, i.e. using the shoulder harness or elbow harness, in addition to the sEMG signal as control input for the prosthetic device.

### BRIEF DESCRIPTION OF THE DRAWINGS

Fig. 1 is a depiction of the hierarchical control structure.
Fig. 2 is a view of the upper level control of the hierarchical controller.
Fig. 3 is a depiction of the lower level control of the hierarchical controller.
Fig. 4 is a depiction of the mechanical input and function of the invention.
Fig. 5 is a view of some of the advanced capabilities of the invention.

### DETAILED DESCRIPTION OF THE PREFERRED EMBODIMENT

The sEMG based control system uses a hierarchical architecture that utilizes two approaches to infer the intended motion for the prosthetic device. The "upper" level control is based on a classification scheme, where a set of sEMG sensor signals allow for categorizing what type of motion is intended by the user. With this information, the set of joints and links that are being controlled are identified. The second layer is the joint motion control layer. The control structure for this layer is such that individual joint positions and motions are directly controlled through a set of dynamic models representing the relationship between the sEMG signal and the joint motion. A second set of models is used to relate the intended finger tip forces with the measured sEMG signals.

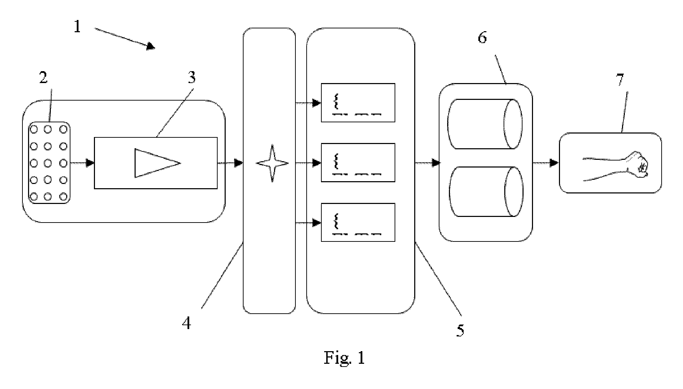

Referring to the invention given in Fig. 1 the components of the hierarchical control architecture are depicted 1. The system has a surface EMG sensor array 2, amplification and filtering section 3, the upper layer control system 4, the lower level control system 5, the activation block 6, and the prosthetic hand device 7.

Referring now to the invention in more detail, in Fig. 1 there is shown a hierarchical controller 1 having five sections, namely the surface EMG sensor array 2, the amplification and filtering section 3, the upper layer control system 4, the lower level control system 5, the activation block 6, and the prosthetic hand mechanism 7. The surface EMG sensor array 2 collects the skin surface electrical potential resulting from the human muscle activation. The sensors combine the signals based on the spatial location where the individual is located with respect to a muscle. The amplification and filtering 3 is performed by using a notch filter removing low and high frequency components from the acquired signal. In addition, in this stage 3, amplification and rectification of the sEMG signal is performed. The upper layer control system 4 is responsible for additional signal processing to format the filtered sEMG signal to a normalized size, which is used to identify what type of motion the prosthetic hand user intends to perform. The lower level control system 5 defines the actual movement of the individual joints of the prosthetic. In addition, the lower level control system 5 is responsible of determining the intended finger forces to be generated by the prosthetic hand device 7. The activation block 6 implements the joint motion and finger force commands by activating motors which drive the joints and generate torques and the resulting finger forces.

In further detail, still referring to the invention of Fig. 1, the surface EMG sensor array 2 is designed by having a measure of an electrical potential between two points on the skin, with respect to a reference point, commonly placed at the outer part of the elbow. The two point measure is on the skin surface in the vicinity of the respective skeletal muscle responsible for a particular motion of a finger or set of fingers. The amplification and filtering 3 is designed by using amplifier circuitry and filtering circuitry. The rectification is performed as well using an amplification circuitry. The upper layer control system 4 and lower level control system 5 are embedded into a microcontroller. The activation block 6 is constructed by using electric motors with cabling to the individual joints of the prosthetic hand device 7.

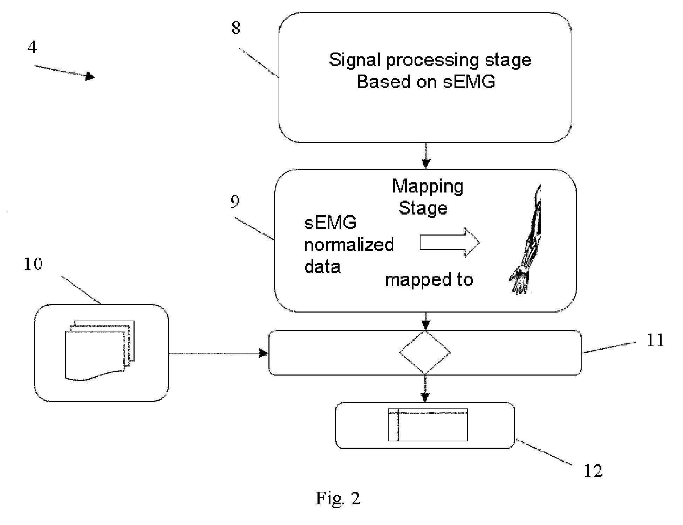

Referring now to Fig. 2, the upper level control 4 is structured by a set of sequential processes. The sEMG signal obtained from the filtering unit 3 is processed by the signal processing stage 8, and mapped by the signal mapping stage 9. The mapped signal is submitted to the compare stage 11 that utilizes a classification table 10. This is then used in the motion set determination 12 stage.

In more detail, still referring to the invention in Fig. 2, the signal processing stage 8 utilizes a number of mathematical operations to break down the filtered, rectified and amplified sEMG signal. The outcome of this process is than normalized in amplitude. The normalized signal is then mapped by the mapping stage 9 to classes of hand and finger motions. The outcome of the signal mapping stage 9 is compared in the compare stage 11 with the classification table 10. Based on the comparison 11, a motion set determination 12 is made.

In further detail, still referring to the invention of Fig. 2, the upper level control 4 is implemented by using a microcontroller. The stages of signal processing 8, mapping 9, compare 11, and motion set determination 12 is programmed in equation form. The classification table 10 is stored in the microcontroller memory in tabular format.

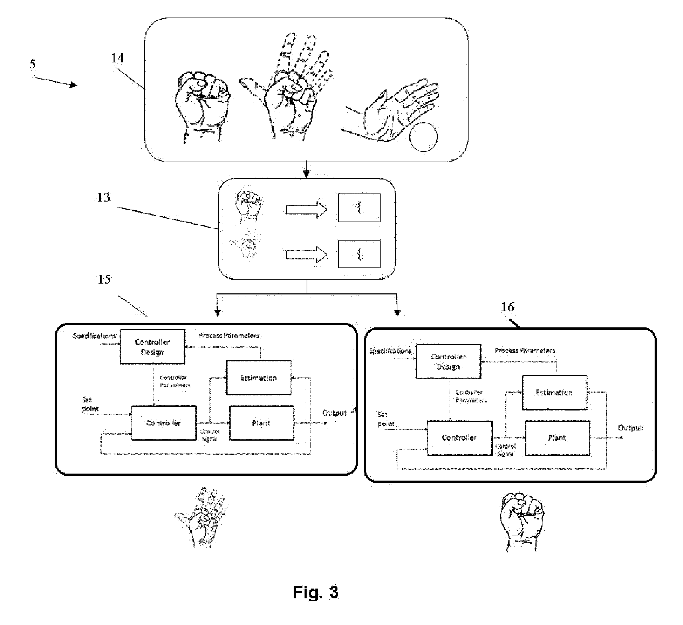

Referring now to Fig. 3, the lower level control 5 is composed of three stages, namely a motion intend stage 14, a model mapping stage 13, and a motion control 15 as well as a force control stage 16.

In more detail, still referring to the invention of Fig. 3, the lower level control system 5 utilizes the information obtained from the upper level control 4 and the resulting motion determination set 12 to determine the motion intend stage 14. This stage 14 determines which joints and which fingers are being controlled. This information is then used in the mapping stage 13 of the lower level control system 5 to map the motions and forces to individual equations. These equations relate the dynamic relationship between the measured and processed 8 sEMG signal and the actual finger motion and finger forces. The finger/joint motions are determined by the motion control stage 15, while the finger forces are determined by the force control stage 16. The motion control stage 15 and the force control stage 16 drive the electric motors of the activation block 6.

In further detail, still referring to the invention of Fig. 3, the lower level control 5 is implemented by using a microcontroller. The stages of motion intend stage 14, the model mapping stage 13, the motion control stage 15 and force control stage 16, are programmed in equation form.

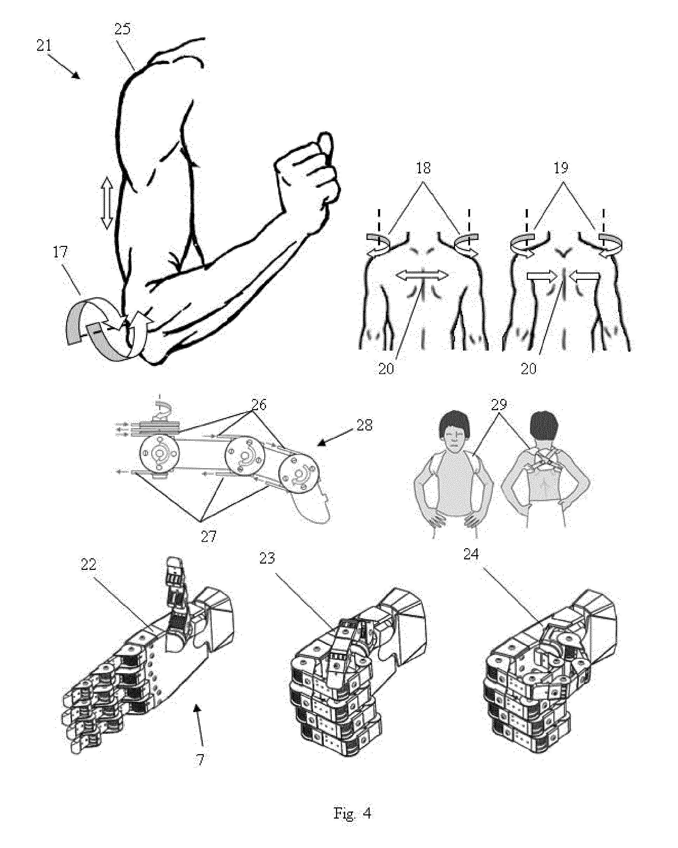

Referring to the invention given in Fig. 4, the primary control input for the mechanical control 21 of the prosthetic hand device 7 are shoulder motions 25 and elbow motions 17. Some of the shoulder motions that are incorporate in the mechanical control are based on protraction 18, and retraction 19, where the shoulder blades 20 move in opposite directions. Some of the resulting prosthetic hand 7 motions are open hand 22, closed hand with thumb up 23, and grip with thumb outside 24. Fig. 4 details the depiction of one of the mechanical fingers 28 of the present invention 7, which contains tension 26 and loosening cables 27.

In more detail, still referring to the invention of Fig. 4, the mechanical control 21 of the prosthetic hand device 7, is based on the motion created when the elbow 17 is rotated. This motion is transferred to the prosthetic hand device 7 via dual cables 26 and 27. These cables create a pull-pull affect that allows one of the cables 26 and 27 to be tensioned independent of the rotation of the elbow 17. When the elbow 17 is rotated in one direction cable 26 is tensioned and cable 27 is loosened. When the elbow 17 is rotated in the opposite direction, cable 27 is tensioned and cable 26 is loosened. Similar control of a different motion set of the invention 7 can be achieved using the shoulder motion 25. As the amputee moves their shoulders 20, the motion is captured with a harness 29 and converted into tension in the Bowden cables 26, 27. The resulting force is then transferred into the designed motion in the prosthetic hand device 7 based on operator input. For example, the operator can go from an open hand 22 to a closed hand with thumb up 23 by moving their shoulders. All simple motions of the prosthetic hand device can be created by the combination of the three input methods, shoulder motion, elbow rotation, and sEMG signal.

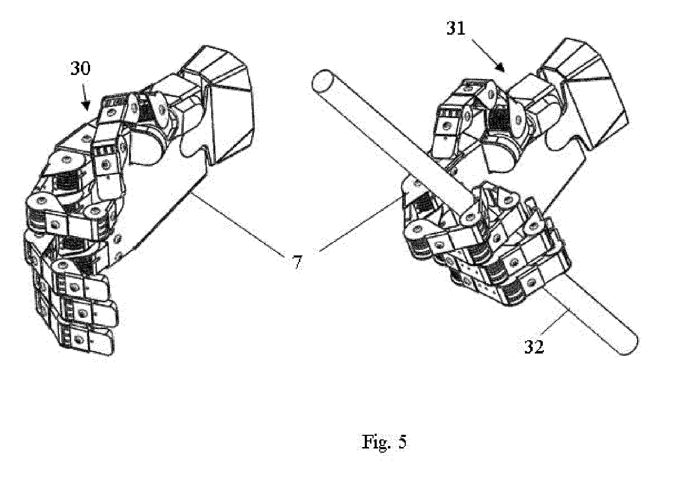

Referring now to Fig. 5, two particular motions of the prosthetic hand device 7 are depicted. The lateral motion 30 and the light tool motion 31 are depicted. The example object for the tool motion is a small cylinder 32.

In more detail, still referring to the invention of Fig. 5, the first motion, lateral 30 is achieved using the hybrid control composed of the hierarchical controller 1 and mechanical controller 21. In the same fashion, the light tool motion 31 can be achieved, i.e. using the hierarchical controller 1 and mechanical controller 21.

While the foregoing written description of the invention enables one of ordinary skill to make and use what is considered presently to be the best mode thereof, those of ordinary skill will understand and appreciate the existence of variations, combinations, and equivalents of the specific embodiment, method, and examples herein. The invention should therefore not be limited by the above described embodiment, method, and examples, but by all embodiments and methods within the scope and spirit of the invention as claimed.

## Claims

1. A hybrid prosthetic hand for an amputee comprising:
an EMG sensor array, said EMG sensor array configured to capture data via sEMG signals,
a microcontroller;
a mechanical controller;
a hierarchical controller;
at least one tension cable;
at least one loosening cable;
at least one Bowden cable;
at least one electric motor;
a harness, wherein said harness is in communication with the amputee;
wherein said mechanical controller is configured to receive input from said harness;
wherein said microcontroller is configured to convert said data from said EMG sensor array into instructions for said hierarchical controller;
wherein said at least one tension cable is strained upon movement of an elbow of the amputee;
mechanical fingers, wherein said mechanical fingers are moved by said Bowden cables via said at least one electric motor when instructed by said hierarchical controller;
wherein said mechanical fingers are moved by said Bowden cables when instructed by said mechanical controller via a shoulder of the amputee; and
wherein all movement of said mechanical fingers is enacted via three input methods selected from the following: motion of the shoulder, rotation of the elbow, sEMG signal.

2. The hybrid prosthetic hand for an amputee of claim 1, wherein said harness is in communication with the shoulder of the amputee.

3. The hybrid prosthetic hand for an amputee of claim 1, wherein said harness is in communication with the elbow of the amputee.

4. The hybrid prosthetic hand for an amputee of claim 1, wherein said at least one electric motor in in communication with said mechanical fingers.

5. The hybrid prosthetic hand for an amputee of claim 1, further comprising:
a motion intend stage;
a model mapping stage;
a motion control stage;
a force control stage;
wherein said motion intend stage, said model mapping stage, said motion control stage, and said force control stage are regulated by said microcontroller;
wherein said motion control stage and said force control stage are configured to determine the extent to which said mechanical fingers move; and
wherein said motion control stage and said force control stage are configured to determine the extent of force applied to said mechanical fingers.

6. The hybrid prosthetic hand for an amputee of claim 5, wherein lateral movement of said harness and said mechanical fingers is achieved via instructions from said hierarchical controller and said mechanical controller.
